# EUROPEAN PATENT SPECIFICATION

(11) **EP 4 331 576 B1**
(45) Date of publication and mention of the grant of the patent: **16.09.2026**
(21) Application number: 22796207.3
(22) Date of filing: 29.04.2022
(51) Int. Cl.: A61K 9/51, A61K 9/00, A61K 31/713, A61K 47/34

(54) **COMPOSITION FOR DRUG DELIVERY COMPRISING NANOPARTICLES NOT CONTAINING AMPHIPHILIC POLYMER**
ZUSAMMENSETZUNG ZUR ARZNEIMITTELABGABE MIT NANOPARTIKELN OHNE AMPHIPHILES POLYMER
COMPOSITION DESTINÉE À L'ADMINISTRATION DE MÉDICAMENT COMPRENANT DES NANOPARTICULES NE CONTENANT PAS DE POLYMÈRE AMPHIPHILE

(30) Priority: 30.04.2021 KR 20210056374
(43) Date of publication of application: 06.03.2024
(73) Proprietor: SAMYANG BIOPHARM CORPORATION, Seongnam-si Gyeonggi-do 13488 (KR)
(72) Inventor: LEE, So Jin, Seoul 02468 (KR); KIM, Go Eun, Seongnam-si Gyeonggi-do 13523 (KR); PARK, Joon Young, Seoul 06622 (KR)
(74) Representative: Wohlfahrt, Jan Günther
(86) International application number: PCT/KR2022/006177
(87) International publication number: WO 2022/231375

(56) References cited:
- EP-A1- 4 079 298
- WO-A1-2018/220553
- WO-A1-2021/101265
- KR-A- 20190 056 045
- US-A1- 2016 228 361
- DEV ASHISH, BINULAL N.S., ANITHA A., NAIR S.V., FURUIKE T., TAMURA H., JAYAKUMAR R.: "Preparation of poly(lactic acid)/chitosan nanoparticles for anti-HIV drug delivery applications", CARBOHYDRATE POLYMERS, vol. 80, no. 3, 5 May 2010 (2010-05-05), GB , pages 833 - 838, XP055980702, ISSN: 0144-8617, DOI: 10.1016/j.carbpol.2009.12.040
- MUNIER, S. ; MESSAI, I. ; DELAIR, T. ; VERRIER, B. ; ATAMAN-ONAL, Y.: "Cationic PLA nanoparticles for DNA delivery: Comparison of three surface polycations for DNA binding, protection and transfection properties", COLLOIDS AND SURFACES B: BIOINTERFACES, vol. 43, no. 3-4, 10 July 2005 (2005-07-10), NL , pages 163 - 173, XP027803477, ISSN: 0927-7765, DOI: 10.1016/j.colsurfb.2005.05.001
- YANG XIAN-ZHU, DOU SHUANG, WANG YU-CAI, LONG HONG-YAN, XIONG MENG-HUA, MAO CHENG-QIONG, YAO YAN-DAN, WANG JUN: "Single-Step Assembly of Cationic Lipid–Polymer Hybrid Nanoparticles for Systemic Delivery of siRNA", ACS NANO, vol. 6, no. 6, 26 June 2012 (2012-06-26), US , pages 4955 - 4965, XP055980704, ISSN: 1936-0851, DOI: 10.1021/nn300500u

## Description

### TECHNICAL FIELD

The present invention relates to a composition for drug delivery, and more specifically, a composition for drug delivery comprising nanoparticles comprising cationic compound and salt of polylactic acid, and not comprising amphiphilic polymer, capable of forming stable nanoparticles without drug, and when a drug added later to the formed nanoparticles, capable of forming stable and effective drug-containing nanoparticles easily, and further increasing the cell delivery efficiency of the drug as compared with nanoparticles comprising amphiphilic polymer.

### BACKGROUND ART

In therapies using anionic drugs including nucleic acid, technologies for safe and efficient drug delivery have been researched for a long time, and various carriers and techniques for delivery have been developed. Carriers are mainly divided into viral carriers utilizing adenovirus, retrovirus or the like, and non-viral carriers utilizing cationic lipid, cationic polymer or the like. Viral carriers are known as being exposed to risks such as non-specific immune response, etc. and having many problems in commercialization due to the complexity of the production process. Thus, recent researches proceed in the direction to improve such disadvantages by using non-viral carriers. In comparison with viral carriers, non-viral carriers have advantages of fewer side effects in terms of *in vivo* safety, and lower production cost in terms of economy.

The representative non-viral carriers for delivering nucleic acid material are a complex of cationic lipid and nucleic acid (lipoplex) and a complex of polycationic polymer and nucleic acid (polyplex). Such a cationic lipid or polycationic polymer stabilizes anionic drug by forming a complex through electrostatic interaction with the anionic drug and increases intracellular delivery, and for these reasons, various researches thereof have been conducted (De Paula D, Bentley MV, Mahato RI, Hydrophobization and bioconjugation for enhanced siRNA delivery and targeting, RNA 13 (2007) 431-56; Gary DJ, Puri N, Won YY, Polymer-based siRNA delivery: Perspectives on the fundamental and phenomenological distinctions from polymer-based DNA delivery, J Control release 121 (2007) 64-73).

In case of personalized vaccine or pandemic situation, once the drug for treatment is determined, it is important to promptly produce and supply nanoparticles containing the drug with minimum process period. Accordingly, it is necessary to develop technology to shorten the process period, and furthermore, to provide ready-made, pre-manufactured nanoparticles regardless of the type of drug.

Nanoparticles containing drug frequently lose stability easily according to the storage environment, and thus they are vulnerable to long-term storage and the quality may be damaged during transportation. In addition, since a complicated production process is necessary for securing sufficient stability, the production is very demanding. Therefore, it has been requested to develop a composition for drug delivery, which is not significantly affected by the storage environment and can be easily used by an end user, and can further increase the cell delivery efficiency of the drug.

### CONTENTS OF THE INVENTION

### PROBLEMS TO BE SOLVED

The purpose of the present invention is to provide a composition for drug delivery, by which drug-containing nanoparticles can be prepared easily and quickly immediately before the use regardless of the kind of drug (e.g., mRNA), and thus once the drug is determined, it can be easily contained in nanoparticles only by simple mixing with the drug, and so the nanoparticle preparation is simple and easy to an end user for use; in addition, even if the drug is changed, for example, to antigen mRNA having different base sequence such as OVA, mTrp2, hTrp2, etc. or the like, it is possible to form stable and effective drug-containing nanoparticles, and thus various drug can be applied to the nanoparticles developed as ready-made product; and furthermore, influence according to the storage or transportation environment can be reduced, facilitating administration to the patient with minimized possibility of drug denaturation and further increased cell delivery efficiency of the drug.

### TECHNICAL MEANS

To achieve the above purpose, the present invention provides a composition for drug delivery comprising nanoparticles, wherein the nanoparticles comprise cationic compound and salt of polylactic acid, and wherein the nanoparticles do not comprise amphiphilic polymer and drug.

In an embodiment, the composition for drug delivery is for intracellular delivery of the drug.

In an embodiment, the drug is selected from nucleic acid, polypeptide, virus or combination thereof.

In an embodiment, the composition for drug delivery further comprises additional solvent.

In an embodiment, the solvent is aqueous solvent, water miscible solvent, or a mixture thereof.

In an embodiment, the composition for drug delivery further comprises one or more additives selected from pH adjusting agent, inorganic salt, saccharide, surfactant, chelating agent, or a combination thereof.

In an embodiment, the composition for drug delivery may be one consisting of: cationic compound; salt of polylactic acid; solvent; and one or more additives selected from pH adjusting agent, inorganic salt, saccharide, surfactant, chelating agent, or a combination thereof.

In an embodiment, the amount of the salt of polylactic acid may be 0.1 to 15 parts by weight, based on 1 part by weight of the cationic compound.

In an embodiment, the amount of the salt of polylactic acid may be 0.5 to 3 parts by weight, based on 1 part by weight of the cationic compound.

In an embodiment, the cationic compound and the salt of polylactic acid may be used for the nanoparticle preparation in the form of solution filtered one or more times.

### EFFECT OF THE INVENTION

The composition for drug delivery according to the present invention is not influenced by the storage or transportation environment, and when using it, an end user can quickly prepare drug-containing nanoparticles, even if the kind of drug (e.g., mRNA) is changed, only by simple mixing of the composition and the drug without complicated process, and accordingly, for example, in case of personalized vaccine or pandemic situation, it is not necessary to optimize the production process with mRNA in hospital, and thus once the complete mRNA is produced, it can be simply mixed with the composition of the present invention and promptly administered into human body. In addition, the composition for drug delivery according to the present invention can further increase the cell delivery efficiency of the drug, as compared with a nanoparticle composition comprising amphiphilic polymer.

### BRIEF EXPLANATION OF THE DRAWINGS

Figure 1 is an image showing the results of agarose gel electrophoresis in the experiment to confirm mRNA-containing nanoparticle preparation by the kit manner conducted in Example A of the present invention.
Figure 2 is an image showing the results of agarose gel electrophoresis in the experiment to confirm mRNA-containing nanoparticle preparation by the non-kit manner (simple mixing) conducted in Example A of the present invention.

### CONCRETE MODE FOR CARRYING OUT THE INVENTION

The present invention will be explained in detail below.

The composition for drug delivery according to the present invention comprises nanoparticles, wherein the nanoparticles comprise cationic compound and salt of polylactic acid, and wherein the nanoparticles do not comprise amphiphilic polymer and drug.

In an embodiment, the particle size of the nanoparticles can be defined by Z-average value, and for example, it may be 800 nm or less, 600 nm or less, 500 nm or less, 400 nm or less, 300 nm or less, 200 nm or less, or 180 nm or less, and also may be 10 nm or more, 50 nm or more, or 100 nm or more. In a concrete embodiment, the particle size of the nanoparticles defined by Z-average value may be, for example, 10 to 800 nm, 10 to 600 nm, 10 to 500 nm, 10 to 400 nm, 10 to 300 nm, 10 to 200 nm, or 10 to 180 nm.

The "Z-average" may mean the average of hydrodynamic diameter of particle distribution measured by using Dynamic light scattering (DSL). The nanoparticles have monodisperse particle distribution, and the polydispersity index thereof may be, for example, 0.01 to 0.30, 0.05 to 0.25, or 0.1 to 0.2.

Also, in an embodiment, the surface charge of the nanoparticles may be, for example, -40 mV or more, -30 mV or more, -20 mV or more, or -10 mV or more, and also may be 40 mV or less, 30 mV or less, 20 mV or less, or 10 mV or less. In a concrete embodiment, the surface charge of the nanoparticles may be, for example, -40 to 40 mV, -30 to 30 mV, -20 to 20 mV, or - 10 to 10 mV. The surface charge may be measured in an environment close to biological environment, and for example, it may be measured in 8 to 12 mM HEPES buffer (pH 7.0 to 7.5).

In case of maintaining the particle size and surface charge of the nanoparticles to the above level, it is preferable in terms of stability of nanoparticle structure, amounts of the components and absorbability in the body, and easiness of sterilization. For example, in case where the drug is nucleic acid, the one or more ends of the nucleic acid may be modified with one or more selected from the group consisting of cholesterol, tocopherol and fatty acids having 10 to 24 carbon atoms. The cholesterol, tocopherol and fatty acids having 10 to 24 carbon atoms include each analogue, derivative and metabolite of the cholesterol, tocopherol and fatty acids.

In a concrete embodiment, the cationic compound may be cationic lipid or cationic polymer, and more concretely, it may be cationic lipid.

In an embodiment, the cationic lipid may may be one or a combination of two or more selected from the group consisting of N,N-dioleyl-N,N-dimethylammoniumchloride (DODAC), N,N-distearyl-N,N-dimethylammoniumbromide (DDAB), N-(1-(2,3-dioleoyloxy)propyl-N,N,N-trimethylammoniumchloride (DOTAP), N,N-dimethyl-(2,3-dioleoyloxy)propylamine (DODMA), N,N,N-trimethyl-(2,3-dioleoyloxy)propylamine (DOTMA), 1,2-diacyl-3-trimethylammonium-propane (TAP), 1,2-diacyl-3-dimethylammonium-propane (DAP), 3(3-[N-(N',N',N'-trimethylaminoethane)carbamoyl]cholesterol (TC-cholesterol), 3β-[N-(N',N'-dimethylaminoethane)carbamoyl] cholesterol (DC-cholesterol), 3β-[N-(N' - monomethylaminoethane)carbamoyl] cholesterol (MC-cholesterol), 3β-[N-(aminoethane)carbamoyl]cholesterol (AC-cholesterol), cholesteryloxypropane-1-amine (COPA), N-(N'-aminoethane)carbamoylpropanoic tocopherol (AC-tocopherol) and N-(N'-methylaminoethane)carbamoylpropanoic tocopherol (MC-tocopherol).

In case of using such a cationic lipid, it is preferable to use polycationic lipid having high cation density in a molecule as less as possible in order to decrease toxicity induced by the cationic lipid, and more concretely, it is preferable to use polycationic lipid wherein the number of the functional group capable of exhibiting positive charge in an aqueous solution is one per molecule.

Accordingly, in a more preferable embodiment, the cationic lipid may be one or more selected from the group consisting of 3β-[N-(N',N',N'-trimethylaminoethane)carbamoyl]cholesterol (TC-cholesterol), 3β-[N-(N',N'-dimethylaminoethane)carbamoyl] cholesterol (DC-cholesterol), 3β-[N-(N'-monomethylaminoethane)carbamoyl]cholesterol (MC-cholesterol), 3β-[N-(aminoethane)carbamoyl]cholesterol (AC-cholesterol), N-(1-(2,3-dioleoyloxy)propyl-N,N,N-trimethylammoniumchloride (DOTAP), N,N-dimethyl-(2,3-dioleoyloxy)propylamine (DODMA) and N,N,N-trimethyl-(2,3-dioleoyloxy)propylamine (DOTMA).

On the other hand, in an embodiment, the cationic polymer may be selected from the group consisting of chitosan, glycol chitosan, protamine, polylysine, polyarginine, polyamidoamine (PAMAM), polyethylenimine, dextran, hyaluronic acid, albumin, high molecular weight polyethylenimine (PEI), polyamine and polyvinylamine (PVAm), and more concretely, it may be one or more selected from the group consisting of polyethylenimine (PEI), polyamine and polyvinylamine (PVAm).

In a concrete embodiment, the cationic lipid may be a cationic lipid of the following Formula 1:

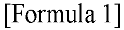

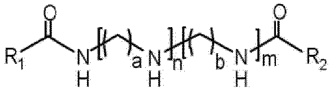

In the above formula,
each of n and m is independently 0 to 12 with the proviso that 2 ≤ n + m ≤ 12;
each of a and b is independently 1 to 6; and
each of R₁ and R₂ is independently selected from the group consisting of saturated and unsaturated hydrocarbon groups having 11 to 25 carbon atoms.

More concretely, in the above formula 1, each of n and m may be independently 1 to 9 with the proviso that 2 ≤ n + m ≤ 10.

More concretely, in the above formula 1, each of a and b may be independently 2 to 4.

More concretely, in the above formula 1, each of R₁ and R₂ may be independently selected from the group consisting of lauryl, myristyl, palmityl, stearyl, arachidyl, behenyl, lignoceryl, cerotyl, myristoleyl, palmitoleyl, sapienyl, oleyl, linoleyl, arachidonyl, eicosapentaenyl, erucyl, docosahexaenyl and cerotyl.

In an embodiment, the cationic lipid may be one or more selected from the group consisting of 1,6-dioleoyl triethylenetetramide(N,N'-((ethane-1,2-diylbis(azanediyl))bis(ethane-2,1-diyl))dioleamide), 1,8-dilinoleoyl tetraethylenepentamide ((9Z,9' Z, 12Z, 12'Z)-N,N'-(((azanediylbis(ethane-2,1-diyl))bis(azanediyl))bis(ethane-2,1-diyl))bis(octadeca-9,12-dienamide)), 1,4-dimyristoleoyl diethylenetriamide ((9Z,9'Z)-N,N'-(azanediylbis(ethane-2,1-diyl))bis(tetradec-9-enamide)), 1,10-distearoyl pentaethylenehexamide (N,N'-(3,6,9,12-tetraazatetradecane-1,14-diyl)distearamide), and 1,10-dioleoyl pentaethylenehexamide (N,N'-(3,6,9,12-tetraazatetradecane-1,14-diyl)dioleamide).

In an embodiment, the salt of polylactic acid is distributed in the core of the nanoparticle, and acts to stabilize the nanoparticle by strengthening the hydrophobicity of the core, and at the same time, to effectively avoid reticuloendothelial system (RES) in the body. That is, the carboxylic anion in the salt of polylactic acid binds to the cationic compound more efficiently than a polylactic acid, and decreases the surface potential of the polymer nanoparticle. Thereby, positive charge of the surface potential of the polymer nanoparticle becomes less than that of a polymer nanoparticle which does not contain a salt of polylactic acid, and thus it may be less captured by reticuloendothelial system and efficiently delivered to target sites (e.g., cancer cells, inflammatory cells, etc.).

In an embodiment, the salt of polylactic acid may have a number average molecular weight of 500 to 50,000 Daltons, and more concretely 1,000 to 10,000 Daltons. If the number average molecular weight of the salt of polylactic acid is less than 500 Daltons, the hydrophobicity becomes too low and thus the salt of polylactic acid may not easily exist at the core (inner wall) of the nanoparticle. If the number average molecular weight of the salt of polylactic acid is greater than 50,000 Daltons, the size of the nanoparticle may become too large.

In an embodiment, the end of the salt of polylactic acid (e.g., sodium salt of polylactic acid) opposite to the end of carboxylic acid-metal (e.g., carboxylic acid-sodium) may be substituted with one selected from the group consisting of hydroxyl, acetoxy, benzoyloxy, decanoyloxy, palmitoyloxy, and alkoxy having 1 to 2 carbon atoms.

In an embodiment, the salt of polylactic acid may be one or more selected from the group consisting of the compounds of the following Formulas 2 to 7 (wherein "COO" means carboxylic group, i.e., "C(=O)O"):

[Formula 2] **RO-CHZ-[A]ₙ-[B]ₘ-COOM**

In Formula 2 above, A is -COO-CHZ-; B is -COO-CHY-, -COO-CH₂CH₂CH₂CH₂CH₂- or -COO-CH₂CH₂OCH₂; R is a hydrogen atom, or acetyl, benzoyl, decanoyl, palmitoyl, methyl or ethyl; each of Z and Y is a hydrogen atom, or methyl or phenyl; M is Na, K or Li; n is an integer of from 1 to 30; and m is an integer of from 0 to 20.

[Formula 3] **RO-CHZ-[COO-CHX]ₚ-[COO-CHY']_{q}-COO-CHZ-COOM**

In Formula 3 above, X is methyl; Y' is a hydrogen atom or phenyl; p is an integer of from 0 to 25, q is an integer of from 0 to 25, with the proviso that p + q is an integer of from 5 to 25; R is a hydrogen atom, or acetyl, benzoyl, decanoyl, palmitoyl, methyl or ethyl; M is Na, K or Li; and Z is a hydrogen atom, methyl or phenyl.

[Formula 4] **RO-PAD-COO-W-M'**

In Formula 4 above, W-M' is 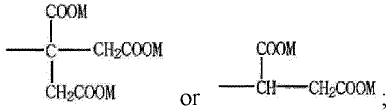 PAD is selected from the group consisting of D,L-polylactic acid, D-polylactic acid, polymandelic acid, copolymer of D,L-lactic acid and glycolic acid, copolymer of D,L-lactic acid and mandelic acid, copolymer of D,L-lactic acid and caprolactone, and copolymer of D,L-lactic acid and 1,4-dioxane-2-one; R is a hydrogen atom, or acetyl, benzoyl, decanoyl, palmitoyl, methyl or ethyl; and M is independently Na, K or Li.

[Formula 5] **S-O-PAD-COO-Q**

In Formula 5 above, S is 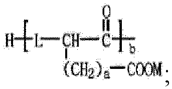 L is -NR₁- or -O-, wherein R₁ is a hydrogen atom or C₁₋₁₀ alkyl; Q is -CH₃, -CH₂CH₃, -CH₂CH₂CH₃, -CH₂CH₂CH₂CH₃, or -CH₂C₆H₅; a is an integer of from 0 to 4; b is an integer of from 1 to 10; M is Na, K or Li; and PAD is one or more selected from the group consisting of D,L-polylactic acid, D-polylactic acid, polymandelic acid, copolymer of D,L-lactic acid and glycolic acid, copolymer of D,L-lactic acid and mandelic acid, copolymer of D,L-lactic acid and caprolactone, and copolymer of D,L-lactic acid and 1,4-dioxane-2-one.

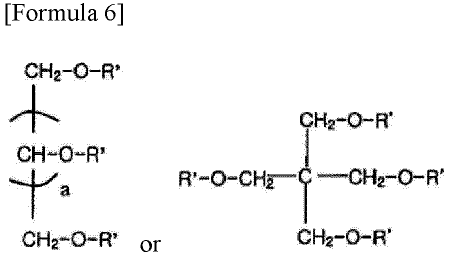

In Formula 6 above, R' is -PAD-O-C(O)-CH₂CH₂-C(O)-OM, wherein PAD is selected from the group consisting of D,L-polylactic acid, D-polylactic acid, polymandelic acid, copolymer of D,L-lactic acid and glycolic acid, copolymer of D,L-lactic acid and mandelic acid, copolymer of D,L-lactic acid and caprolactone, and copolymer of D,L-lactic acid and 1,4-dioxane-2-one, M is Na, K or Li; and a is an integer of from 1 to 4.

[Formula 7] **YO-[-C(O)-(CHX)ₐ-O-]ₘ-C(O)-R-C(O)-[-O-(CHX')_{b}-C(O)-]ₙ-OZ**

In Formula 7 above, X and X' are independently hydrogen, C₁₋₁₀ alkyl or C₆₋₂₀ aryl; Y and Z are independently Na, K or Li; m and n are independently an integer of from 0 to 95, with the proviso that 5 < m + n < 100; a and b are independently an integer of from 1 to 6; and R is -(CH₂)ₖ-, C₂₋₁₀ divalent alkenyl, C₆₋₂₀ divalent aryl or a combination thereof, wherein k is an integer of from 0 to 10.

In an embodiment, the salt of polylactic acid may be a compound of Formula 2 or Formula 3.

In an embodiment, the amount of the salt of polylactic acid in the nanoparticle comprised in the composition for drug delivery of the present invention may be 0.1 part by weight or more, 0.2 part by weight or more, 0.3 part by weight or more, 0.4 part by weight or more, or 0.5 part by weight or more, and 15 parts by weight or less, 10 parts by weight or less, 9 parts by weight or less, 8 parts by weight or less, 7 parts by weight or less, 6 parts by weight or less, 5 parts by weight or less, 4 parts by weight or less, or 3 parts by weight or less, based on 1 part by weight of the cationic compound.

More concretely, the amount of the salt of polylactic acid may be 0.1 to 15 parts by weight, still more concretely 0.2 to 10 parts by weight, still more concretely 0.3 to 5 parts by weight, and still more concretely 0.5 to 3 parts by weight, based on 1 part by weight of the cationic compound.

The composition for drug delivery of the present invention can easily form drug-containing nanoparticles only by simple mixing with the drug. The term "simple mixing" can include all actions of "mixing," and it means that the action of mixing is not subject to any specific conditions. The mixing can be done in various manners such as dropping, vortexing, decanting, etc., but it is not limited thereto. According to an embodiment, in case of using the composition for drug delivery of the present invention, drug-containing nanoparticles can be formed rapidly, for example, within 1 minute, within 30 seconds, or within 15 seconds, in an amount of 90% or more, 95% or more, or 99% or more of the amount that can be formed theoretically.

In an embodiment, the cationic compound and the salt of polylactic acid form nanoparticles through electrostatic interaction, and an end user can form drug-containing nanoparticles by simply mixing the formed nanoparticles and the drug. Thus, according to an embodiment, the drug-containing nanoparticles prepared by mixing the composition for drug delivery of the present invention with the drug can be in a form where at least a part of the drug is combined to the outside of the nanoparticle. Such a drug-containing nanoparticle structure improves stability of the drug in blood or body fluid.

In an embodiment, the drug may be selected from nucleic acid, polypeptide, virus or combination thereof.

The "nucleic acid" can be, for example, DNA, RNA, siRNA, shRNA, miRNA, mRNA, aptamer, antisense oligonucleotide, or a combination thereof, but it is not limited thereto.

The "polypeptide" may mean a protein having activity in the body such as antibody or fragment thereof, cytokine, hormone or analog thereof, or a protein that can be recognized as antigen through a series of processes in the body, including polypeptide sequence of antigen, analog or precursor thereof.

The "virus" may be oncolytic virus, and for example, may be one or more selected from the group consisting of adenovirus, vaccinia virus, herpes simplex virus (HSV) and vesicular stomatitis virus (VSV). In an embodiment, the oncolytic virus is adenovirus. The adenovirus used in the concrete example of the present invention comprises luciferase gene, and it can be confirmed through imaging.

The virus can express several kinds of treatment genes in the body of the subject, and it is not limited in terms of specific molecular weight, protein, bioactivity or field of treatment. The virus for prevention can induce immunity to the target disease in the body of the subject. Nanoparticles comprising virus for preventing disease can reduce immunity induction due to the virus itself, designate or extend the target cell, and reduce hyperimmune reaction to the virus when administered again and thereby provide advantage of obtaining available effect by inoculation for several times.

The amount of the drug may be, based on the total weight of the drug-containing nanoparticle prepared by mixing with the composition for drug delivery of the present invention, for example, 30 % by weight or less, 25 % by weight or less, 20 % by weight or less, 15 % by weight or less, 10 % by weight or less, or 5 % by weight or less, and also may be 0.001 % by weight or more, 0.01 % by weight or more, 0.05 % by weight or more, 0.1 % by weight or more, 0.25 % by weight or more, 0.5 % by weight or more, or 1 % by weight or more. In a concrete embodiment, the amount of the drug may be, based on the total weight of the drug-containing nanoparticle, for example, 0.05 to 30 % by weight, 0.1 to 25 % by weight, 0.25 to 20 % by weight, 0.5 to 15 % by weight, 1 to 10 % by weight, or 1 to 5 % by weight. If the amount of the drug based on the total weight of the drug-containing nanoparticle is less than the above range, the amount of nanoparticle used as delivery carrier becomes too much as compared with the drug, and thus there may be a side effect due to the nanoparticle delivery carrier. If the amount of the drug is greater than the above range, the size of the drug-containing nanoparticle becomes too large, and thus the particle stability may be lowered and the rate of loss during filter sterilization may increase. In case where the drug is virus, the drug-containing nanoparticle may comprise virus 1x10⁶ to 1x10¹⁴ VP (Virus particle), 1x10⁷ to 1x10¹³ VP, 1x10⁸ to 1x10¹² VP, or 1x10⁹ to 1x10¹¹ VP.

The amount of the salt of polylactic acid in the nanoparticle comprised in the composition for drug delivery of the present invention may be adjusted according to the drug. For example, in case where the drug is virus, the amount of the salt of polylactic acid may be 3 to 15 parts by weight, based on 1 part by weight of the cationic compound, and in other embodiment, in case where the drug is nucleic acid, the amount of the salt of polylactic acid may be 0.2 to 10 parts by weight, 0.3 to 5 parts by weight, or 0.5 to 3 parts by weight, based on 1 part by weight of the cationic compound.

The amount of the cationic compound in the drug-containing nanoparticle prepared by mixing the composition for drug delivery of the present invention with the drug may be, based on 1 part by weight of the drug, for example, 25 parts by weight or less, 20 parts by weight or less, 18 parts by weight or less, 15 parts by weight or less, 12 parts by weight or less, 10 parts by weight or less, or 8 parts by weight or less, and also may be 1 part by weight or more, 1.5 parts by weight or more, 2 parts by weight or more, 2.5 parts by weight or more, 3 parts by weight or more, or 3.5 parts by weight or more. In an embodiment, the amount of the cationic compound in the drug-containing nanoparticle may be, based on 1 part by weight of the drug, 1 to 25 parts by weight, 1.5 to 10 parts by weight, 2 to 15 parts by weight, 2.5 to 10 parts by weight, or 3 to 8 parts by weight. Also, in case where the drug is virus, more concretely adenovirus, the amount of the cationic compound may be, based on virus 1x10¹⁰ VP, 1 µg or more, 5 µg or more, 10 µg or more, 15 µg or more, or 18 µg or more, and also may be 150 µg or less, 100 µg or less, 50 µg or less, or 30 µg or less, and for example, it may be 1 µg to 150 µg, 5 µg to 100 µg, 10 µg to 50 µg, or 15 µg to 30 µg. If the amount of the cationic compound in the drug-containing nanoparticle is less than the above range, the drug may not be contained stably in the nanoparticle. If the amount of the cationic compound is greater than the above range, the size of the drug-containing nanoparticle becomes too large, and thus the particle stability may be lowered and the rate of loss during filter sterilization may increase.

In case where the drug is nucleic acid, the cationic compound and the nucleic acid are combined together through electrostatic interaction. In an embodiment, the ratio of quantities of electric charge of the cationic compound (N) and the nucleic acid (P) (N/P: the ratio of the positive electric charge of the cationic compound to the negative electric charge of the nucleic acid) may be 0.5 or more, 1 or more, 2 or more, or 3.5 or more, and also may be 100 or less, 50 or less, or 20 or less, and for example, it may be 0.5 to 100, 1 to 50, 2 to 20, 5 to 15, or 7 to 12. If the ratio (N/P) is less than the above range, a sufficient amount of the nucleic acid may not be contained in the nanoparticle. If the ratio (N/P) is greater than the above range, toxicity may be induced. In addition, the N/P ratio may act importantly for expression of the effective ingredient specifically in spleen.

The nanoparticles comprised in the composition for drug delivery of the present invention do not comprise amphiphilic polymer (for example, amphiphilic block copolymer).

More concretely, the nanoparticles comprised in the composition for drug delivery of the present invention do not comprise any amphiphilic block copolymer which is an A-B type block copolymer comprising a hydrophilic A block (for example, one or more selected from the group consisting of polyalkylene glycol, polyvinyl alcohol, polyvinyl pyrrolidone, polyacrylamide, and derivatives thereof) and a hydrophobic B block (for example, one or more selected from the group consisting of polyester, polyanhydride, polyamino acid, polyorthoester and polyphosphazine).

In an embodiment, when mixed with drug (e.g., mRNA), in order to increase the intracellular transduction efficiency thereof, the nanoparticles comprised in the composition for drug delivery of the present invention may further comprise fusogenic lipid.

In an embodiment, the amount of the fusogenic lipid comprised in the nanoparticles comprised in the composition for drug delivery of the present invention may be 0.01 to 50 % by weight, more concretely 0.1 to 10 % by weight, based on total weight of the drug-containing nanoparticle prepared by mixing with drug (e.g., mRNA).

In an embodiment, the fusogenic lipid is combined with the cationic compound by hydrophobic interaction to form nanoparticle structure.

In an embodiment, the fusogenic lipid may be one or a combination of two or more selected from the group consisting of phospholipid, cholesterol and tocopherol.

More concretely, the phospholipid may be one or more selected from the group consisting of phosphatidylethanolamin (PE), phosphatidylcholine (PC) and phosphatidic acid. The phosphatidylethanolamin (PE), phosphatidylcholine (PC) and phosphatidic acid may be in a form combined with one or two C₁₀₋₂₄ fatty acids. The cholesterol and tocopherol include analogues, derivatives and metabolites of each of the cholesterol and tocopherol.

Still more concretely, the fusogenic lipid may be one or a combination of two or more selected from the group consisting of dilauroyl phosphatidylethanolamine, dimyristoyl phosphatidylethanolamine, dipalmitoyl phosphatidylethanolamine, distearoyl phosphatidylethanolamine, dioleoyl phosphatidylethanolamine (DOPE), 1,2-dipalmitoleoyl-sn-glycero-3-phosphoethanolamine (DPPE), dilinoleoyl phosphatidylethanolamine, 1-palmitoyl-2-oleoyl phosphatidylethanolamine, 1,2-diphytanoyl-3-sn-phosphatidylethanolamine, 1,2-dipalmitoleoyl-sn-glycero-3-phosphocholine (DPPC), 1,2-dioleoyl-sn-glycero-3-phosphocholine (DOPC), dilauroyl phosphatidylcholine, dimyristoyl phosphatidylcholine, dipalmitoyl phosphatidylcholine, distearoyl phosphatidylcholine, dioleoyl phosphatidylcholine, dilinoleoyl phosphatidylcholine, 1-palmitoyl-2-oleoyl phosphatidylcholine, 1,2-diphytanoyl-3-sn-phosphatidylcholine, dilauroyl phosphatidic acid, dimyristoyl phosphatidic acid, dipalmitoyl phosphatidic acid, distearoyl phosphatidic acid, dioleoyl phosphatidic acid, dilinoleoyl phosphatidic acid. 1-palmitoyl-2-oleoyl phosphatidic acid, 1,2-diphytanoyl-3-sn-phosphatidic acid, cholesterol and tocopherol.

Still more concretely, the fusogenic lipid may be one or more selected from the group consisting of dioleoyl phosphatidylethanolamine (DOPE), 1,2-dipalmitoleoyl-sn-glycero-3-phosphocholine (DPPC), 1,2-dioleoyl-sn-glycero-3-phosphocholine (DOPC) and 1,2-dipalmitoleoyl-sn-glycero-3-phosphoethanolamine (DPPE).

In an embodiment, the composition for drug delivery of the present invention may further comprise aqueous solution, water miscible organic solvent, or a combination thereof. The "aqueous solution" may be used for the same meaning as water solution, and may mean, for example, water, sterilized water, buffer solution, injection solution, etc., and it may be a buffer solution further comprising organic acid. The aqueous solution may be, for example, citric acid buffer, PBS buffer, etc., but it is not limited thereto. The "water miscible organic solvent" may be C1 to C4 lower alcohol, acetone, acetonitrile, a water mixture thereof or a mixture thereof, but it is not limited thereto.

In an embodiment, the cationic compound and the salt of polylactic acid may be used for the nanoparticle preparation in the form of solution filtered one or more times. More concretely, the filtering may be conducted by using a hydrophilic filter. The material of the hydrophilic filter may be, for example, nylon, mixed cellulose ester (MCE), polyethylsulfone (PES), polyvinylidene difluoride (PVDF), cellulose acetate (CA), polytetrafluoroethylene (PTFE), or a mixture thereof, but it is not limited thereto. In case of being subjected to hydrophilic filtering, the drug may be included in nanoparticle more successfully, and the stability of the drug-containing nanoparticle may increase.

In an embodiment, the composition for drug delivery of the present invention may further comprise a stabilizing agent suitable for improving stability of the drug. The stabilizing agent may include pH adjusting agent, inorganic salt, saccharide, surfactant, chelating agent, etc., but it is not limited thereto. The "saccharide" may mean monosaccharide, disaccharide, sugar alcohol which is reduced sugar thereof, and polymer of single or mixed polysaccharides, etc., and the polysaccharide may mean tri- or more saccharide. For example, the monosaccharide may be mannose, glucose, arabinose, fructose, galactose, etc.; the disaccharide may be sucrose, trehalose, maltose, lactose, cellobiose, gentiobiose, isomaltose, melibose, etc.; the sugar alcohol may be mannitol, sorbitol, xylitol, erythritol, maltitol, etc.; and the polysaccharide may be raffinose, dextran, starch, hydroxyethyl starch, cyclodextrin, cellulose, hetastarch, oligosaccharide, etc. but it is not limited thereto. The "pH adjusting agent" may be Tris, glycine, histidine, glutamate, succinate, phosphate, acetate, aspartate, or a combination thereof, and the "surfactant" may be sodium lauryl sulfate, dioctyl sodium sulfosuccinate, dioctyl sodium sulfonate, chenodeoxycholic acid, N-lauroylsarcosine sodium salt, lithium dodecyl sulfate, 1-octanesulfonic acid sodium salt, sodium cholate hydrate, sodium deoxycholate, glycodeoxycholic acid sodium salt, benzalkonium chloride, Triton X-100, Triton X-114, lauromacrogol 400, polyoxyl 40 stearate, polysorbate 20, 40, 60, 65 or 80, or a combination thereof, but it is not limited thereto. The "chelating agent" may be citric acid, polyphenolic acid, EDTA, DTPA, EDDHA, or a combination thereof, but it is not limited thereto. The "inorganic salt" means a salt of monovalent or divalent metal and may be NaCl, KCl, MgCl₂, CaCl₂, MgSO₄, CaSO₄, CaCO₃, MgCO₃, etc., but it is not limited thereto.

For example, in case where the drug to be mixed with is virus, the composition for drug delivery of the present invention may further comprise 5 to 15 mM Tris, 5 to 15 mM histidine, 50 to 90 mM NaCl, 2 to 8% (w/v) sucrose, 0.5 to 1.5 mM MgCl₂, 0.005 to 0.05% (w/v) PS-80, 0.05 to 0.15 mM EDTA, and 0.1 to 1.0% (v/v) ethanol, and the pH may be 7.0 to 8.0. In other embodiment, in case where the drug to be mixed with is nucleic acid, the second chamber may further comprise PBS buffer, for example, a solution comprising 2.0 to 3.5 mM KCl, 1.0 to 2.5 mM KH₂PO₄, 125 to 145 mM NaCl and 7.5 to 9.5 mM Na₂HPO₄ with PH 7.0 to pH 8.0.

In an embodiment, the composition for drug delivery of the present invention may be one consisting of: the cationic compound; the salt of polylactic acid; solvent; and one or more additives selected from pH adjusting agent, inorganic salt, saccharide, surfactant, chelating agent, or a combination thereof.

In an embodiment, the particle size of the drug-containing nanoparticles prepared by mixing the composition for drug delivery of the present invention and the drug can be defined by Z-average value, and for example, it may be 800 nm or less, 600 nm or less, 500 nm or less, or 400 nm or less, and also may be 100 nm or more, 150 nm or more, 200 nm or more, or 250 nm or more. In a concrete embodiment, the particle size of the drug-containing nanoparticles defined by Z-average value may be, for example, 100 to 800 nm, 100 to 600 nm, 100 to 500 nm, or 100 to 400 nm.

The present invention will be explained below in more detail with reference to the following Examples.

### EXAMPLES

### [Example A]

### (1) Preparation of nanoparticle composition

As an example, the nanoparticle composition was prepared by using 1,6-dioleoyl triethylenetetramide (dioTETA) and sodium salt of polylactic acid (PLANa). Concretely, dioTETA was dissolved in 20 mM sodium acetate buffer to prepare dioTETA solution with 5 mg/mL concentration, and PLANa was dissolved in water to prepare PLANa solution with 10 mg/mL concentration, and then each of the solutions was filtered and sterilized by using 0.22 µm hydrophilic filter, and taken and mixed together so that the dioTETA/PLANa mole ratio was 1, and the final volume was adjusted with water.

As a comparative example, the nanoparticle composition was prepared by mixing a cationic lipid (SM102), distearoylphosphatidylcholine (DSPC), cholesterol, and 1,2-dimyristoyl-rac-glycero-3-methoxypolyethylene glycol-2000 (DMG-PEG 2000).

### (2) Preparation of mRNA solution

The mRNA solution was prepared by diluting GFP mRNA with PBS. In preparing mRNA-containing nanoparticles, 5 µg of mRNA was used.

The nanoparticle composition and the mRNA solution prepared above were mixed by vortexing to prepare mRNA-containing nanoparticles.

### (3) Experiment to confirm mRNA-containing nanoparticle preparation

In order to confirm that nanoparticles and mRNA were combined normally when the nanoparticle composition and the mRNA solution were mixed, agarose gel electrophoresis was conducted. For each of Example and Comparative Example, after the nanoparticle composition and the mRNA solution were mixed simply, a volume corresponding to 400 ng of mRNA was taken therefrom and mixed with loading dye. Then, it was loaded on 1% agarose gel and subjected to electrophoresis with suitable voltage and time, and the mRNA band image was observed through UV transilluminator. At that time, Dyne Gel Safe Red Kit (Dyne Bio) was used as nucleic acid dye.

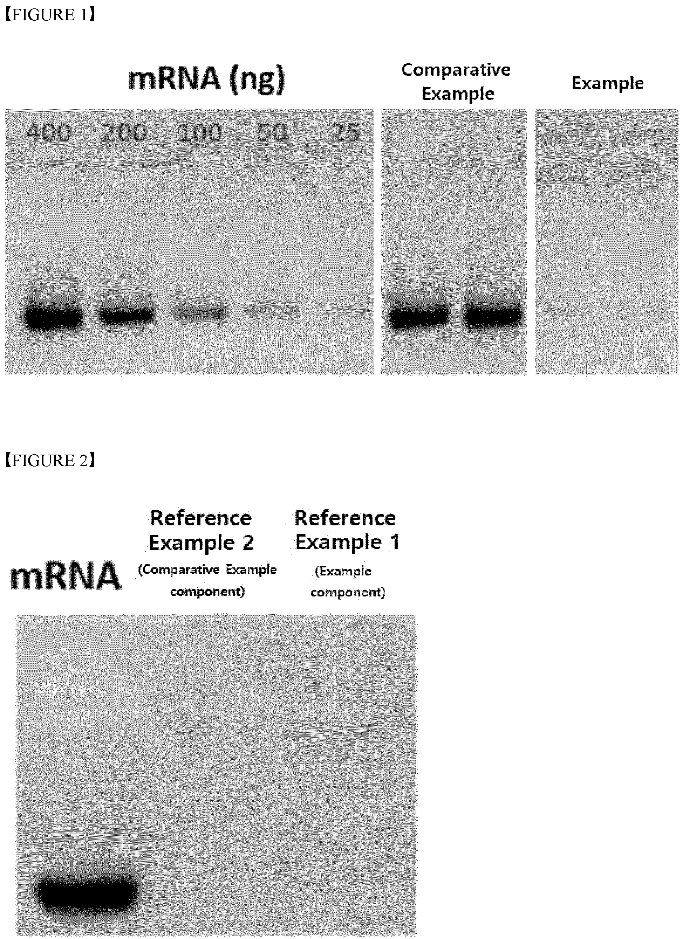

The results of the measurement are shown in Figure 1. According to Figure 1, in Example, the exposed mRNA band was hardly shown due to the high bonding force between the nanoparticle of the first chamber and mRNA, whereas in Comparative Example, most of mRNA was observed since the nanoparticle of the first chamber could not bind with mRNA. That is, nanoparticles containing mRNA were prepared successfully in Example, but not in Comparative Example.

As Reference, the components used in each of the above Example and Comparative Example for mRNA-containing nanoparticle preparation were mixed altogether at one time, and agarose gel electrophoresis was conducted in the same manner as above.

The results of the measurement are shown in Figure 2. According to Figure 2, in both of Reference Example 1 using the components of Example and Reference Example 2 using the components of Comparative Example, the exposed mRNA band was hardly shown. That is, in case of using the components of Comparative Example, preparation of mRNA-containing nanoparticles was possible by simple mixing of all components, but impossible by preparing each of the nanoparticle composition and the mRNA solution, and mixing them. This means that in case of using the components of Comparative Example, it is not possible to obtain the advantage according to the present invention (i.e., the advantage that there is no influence by the storage or transportation environment, and an end user can quickly prepare drug-containing nanoparticles without complicated process even if the kind of drug is changed).

### [Example B]

### (1) Preparation of nanoparticle composition

As an example, the nanoparticle composition was prepared by using 1,6-dioleoyl triethylenetetramide (dioTETA) and sodium salt of polylactic acid (PLANa). Concretely, dioTETA was dissolved in 20 mM sodium acetate buffer to prepare dioTETA solution with 5 mg/mL concentration, and PLANa was dissolved in water to prepare PLANa solution with 10 mg/mL concentration, and then each of the solutions was filtered and sterilized by using 0.22 µm hydrophilic filter. Meanwhile, sucrose was dissolved in water to prepare sucrose solution with 400 mg/mL concentration. The solutions were taken and mixed together so that the dioTETA/PLANa mole ratio was 1, and 10% sucrose solution was added thereto for 10% of the final volume.

### (2) Preparation of mRNA solution

The mRNA solution was prepared by diluting GFP mRNA with PBS. In preparing mRNA-containing nanoparticles, 5 µg of mRNA was used.

### (3) Experiment for storage stability of mRNA-containing nanoparticles

The above nanoparticle composition was stored frozen at ultra-low temperature for 1 to 4 days and then thawed at room temperature, and mixed with the mRNA solution by vortexing to prepare mRNA-containing nanoparticles (Example). Meanwhile, the mRNA-containing nanoparticle sample prepared by mixing the nanoparticle composition and the mRNA solution was stored frozen, and after 1 to 4 days, it was thawed at room temperature (Comparative Example). Also, the above nanoparticle composition was stored frozen at ultra-low temperature for 1 to 4 days and then thawed at room temperature, and the thawed nanoparticle sample (not containing mRNA) was used as Control.

For each nanoparticle sample of the above Example, Comparative Example and Control, the particle size was measured through Dynamic light scattering (DLS), and the results are shown in Table 1.

**[Table 1]**

| **Days of frozen storage** | **Z-average particle size (nm)** | | |
|---|---|---|---|
| | **Control** | **Example** | **Comparative Example** |
| **0 day** | 154.0 | 330.1 | 330.1 |
| **1 day** | 152.5 | 283.8 | 1337.0 |
| **2 days** | 149.4 | 270.0 | 1396.0 |
| **3 days** | 162.2 | 296.7 | 1157.0 |
| **4 days** | 162.0 | 315.3 | 1152.3 |

As shown in Table 1 above, the particle size of the nanoparticles stored frozen for 1 to 4 days and then thawed at room temperature was maintained similar to that before freezing (Control), and the particle size of the mRNA-containing nanoparticles, which was obtained by mixing the nanoparticles stored frozen for 1 to 4 days and then thawed at room temperature with mRNA, was maintained similar to that of mRNA-containing nanoparticles obtained by using the nanoparticles before freezing (Example). Whereas, the particle size of the nanoparticles stored frozen in a state of containing mRNA for 1 to 4 days and then thawed at room temperature was increased, and particle precipitation was observed (Comparative Example). That is, according to the present invention, even after long term storage, the particle size of the nanoparticles can be maintained similar to that before the storage, and only the simple mixing thereof with drug (for example, mRNA) can prepare mRNA-containing nanoparticles, and thus eventually the storage stability of mRNA-containing nanoparticles can be improved remarkably.

### [Example C]

### (1) Preparation of nanoparticle composition

For examples, the nanoparticle composition was prepared by using 1,6-dioleoyl triethylenetetramide (dioTETA) and sodium salt of polylactic acid (PLANa). Concretely, dioTETA was dissolved in 20 mM sodium acetate buffer to prepare dioTETA solution with 5 mg/mL concentration, and PLANa was dissolved in water to prepare PLANa solution with 10 mg/mL concentration, and then each of the solutions was filtered and sterilized by using 0.22 µm hydrophilic filter, and taken and mixed together with the ratio shown in Table 2.

For comparative examples, each of sodium salt of polylactic acid (PLANa) and monomethoxypolyethylene glycol-polylactide copolymer (mPEG-PLA) was dissolved in water to prepare each solution of PLANa/mPEG-PLA with 10 mg/mL concentration, and then each of the solutions was filtered and sterilized by using 0.22 µm hydrophilic filter, and taken and mixed together with the ratio shown in Table 2 to prepare the nanoparticle composition.

**[Table 2]**

| | **dioTETA** | **mPEG-PLA** | **Salt of polylactic acid** |
|---|---|---|---|
| **Example 1** | | - | 9.5 µg |
| **Example 2** | | | 19.1 µg |
| **Example 3** | | | 28.6 µg |
| **Example 4** | 15 µg | | 38.2 µg |
| **Comparative Example 1** | | 41.6 µg | 9.5 µg |
| **Comparative Example 2** | | | 19.1 µg |
| **Comparative Example 3** | | | 28.6 µg |
| **Comparative Example 4** | | | 38.2 µg |

### (2) Preparation of mRNA solution

The mRNA solution was prepared by diluting luciferase mRNA with PBS. In preparing mRNA-containing nanoparticles, 5 µg of mRNA was used.

The nanoparticle composition and the mRNA solution prepared above were mixed by vortexing to prepare mRNA-containing nanoparticles.

### (3) Experiment for evaluating intracellular transduction efficiency of mRNA-containing nanoparticles

In order to compare and evaluate the intracellular transduction efficiency of mRNA-containing nanoparticles according to the presence/absence of mPEG-PLA, HepG2 (human liver cancer) cell line was treated with the nanoparticles and the transduction efficiency was measured. That is, the mRNA-containing nanoparticles of Examples 1 to 4 and Comparative Examples 1 to 4 were aliquoted to the HepG2 cell sown on 96-well plate in an amount corresponding to 250 ng mRNA per well. After 15 to 24 hours, the amount of luciferase expressed from the cell (luminescence, RLU) was measured by using ONE-GloTM Luciferase Assay System (Promega). As Control, a conventionally used transfection reagent, TransIT^{®}-mRNA Transfection Kit (Mirus Bio), was used. The measurement results are shown in Table 3.

**[Table 3]**

| | **Luminescence, RLU** |
|---|---|
| **Example 1** | 8.5x10⁵ |
| **Example 2** | 1.0x10⁶ |
| **Example 3** | 1.1x10⁶ |
| **Example 4** | 3.0x10⁵ |
| **Comparative Example 1** | 1.5x10⁵ |
| **Comparative Example 2** | 5.0x10⁴ |
| **Comparative Example 3** | 3.0x10⁴ |
| **Comparative Example 4** | 3.0x10⁴ |
| **Control** | 8.0x10⁴ |

As shown in Table 3 above, all of the mRNA-containing nanoparticles of Examples exhibited remarkably higher intracellular transduction efficiency than those of Comparative Examples and Control.

### [Example D]

### (1) Preparation of nanoparticle composition

The nanoparticle composition was prepared by using 1,6-dioleoyl triethylenetetramide (dioTETA) and sodium salt of polylactic acid (PLANa). Concretely, dioTETA was dissolved in 20 mM sodium acetate buffer to prepare dioTETA solution with 5 mg/mL concentration, and PLANa was dissolved in water to prepare PLANa solution with 10 mg/mL concentration, and then each of the solutions was filtered and sterilized by using 0.22 µm hydrophilic filter, and taken and mixed together so that the dioTETA/PLANa mole ratio was 1, and the final volume was adjusted with water.

### (2) Preparation of mRNA solution

The mRNA solutions were prepared by diluting each of three (3) kinds of mRNA (human Trp2, murine Trp2, OVA mRNA) with PBS. In preparing mRNA-containing nanoparticles, 10 µg of mRNA was used.

The nanoparticle composition and the mRNA solution prepared above were mixed by vortexing to prepare mRNA-containing nanoparticles.

### (3) Experiment to confirm mRNA-containing nanoparticle preparation according to the kind of mRNA

In order to confirm that nanoparticles and mRNA were combined normally when the nanoparticle composition and the mRNA solution were simply mixed even though the kinds of mRNA are different, DLS (Dynamic Light Scattering) analysis was conducted. Three (3) kinds of mRNA-containing nanoparticles were prepared by simply mixing the nanoparticle composition and the solution containing each of the three (3) kinds of mRNA with different sizes and base sequences (OVA: 1437nt, hTrp2: 990nt, mTrp2: 1815nt). For measuring the particle size through DLS analysis, a volume corresponding to 500 ng of mRNA was taken and diluted with PBS reagent, and placed in a dedicated cuvette for the measurement. For measuring the surface potential, the same volume was taken and diluted with 10mM HEPES reagent, and placed in a dedicated cuvette for the measurement. The measurement results are shown in Table 4.

**[Table 4]**

| **mRNA** | **Z-average particle size (nm)** | **Surface potential (Zeta potential, mV)** |
|---|---|---|
| hTrp2 mRNA | 366.6 | -13.8 |
| mTrp2 mRNA | 367.7 | -15.7 |
| OVA mRNA | 332.1 | -13.8 |

As shown in Table 4 above, all of the three (3) kinds mRNA formed mRNA-containing nanoparticles with similar particle size in a range of from 330 to 370 nm and surface potential in a range of from -10 to -20 mV.

## Claims

1. A composition for drug delivery comprising nanoparticles,
wherein the nanoparticles comprise cationic compound and salt of polylactic acid, and
wherein the nanoparticles do not comprise amphiphilic polymer and drug.

2. The composition for drug delivery of Claim 1, wherein the composition is for intracellular delivery of drug.

3. The composition for drug delivery of Claim 2, wherein the drug is selected from nucleic acid, polypeptide, virus or combination thereof.

4. The composition for drug delivery of Claim 1, further comprising additional solvent.

5. The composition for drug delivery of Claim 4, wherein the solvent is aqueous solvent, water miscible solvent, or a mixture thereof.

6. The composition for drug delivery of Claim 1, further comprising one or more additives selected from pH adjusting agent, inorganic salt, saccharide, surfactant, chelating agent, or a combination thereof.

7. The composition for drug delivery of Claim 1, which consists of: cationic compound; salt of polylactic acid; solvent; and one or more additives selected from pH adjusting agent, inorganic salt, saccharide, surfactant, chelating agent, or a combination thereof.

8. The composition for drug delivery of Claim 1, wherein the amount of the salt of polylactic acid is 0.1 to 15 parts by weight, based on 1 part by weight of the cationic compound.

9. The composition for drug delivery of Claim 1, wherein the amount of the salt of polylactic acid is 0.5 to 3 parts by weight, based on 1 part by weight of the cationic compound.

10. The composition for drug delivery of Claim 1, wherein the cationic compound and the salt of polylactic acid are used for the nanoparticle preparation in the form of solution filtered one or more times.

## Patentansprüche

1. Zusammensetzung zur Wirkstoffabgabe, umfassend Nanopartikel,
wobei die Nanopartikel eine kationische Verbindung und ein Salz der Polymilchsäure umfassen und
wobei die Nanopartikel kein amphiphiles Polymer und keinen Wirkstoff umfassen.

2. Zusammensetzung zur Wirkstoffabgabe nach Anspruch 1, wobei die Zusammensetzung zur intrazellulären Abgabe eines Wirkstoffs bestimmt ist.

3. Zusammensetzung zur Wirkstoffabgabe nach Anspruch 2, wobei der Wirkstoff ausgewählt ist aus Nukleinsäure, Polypeptid, Virus oder einer Kombination davon.

4. Zusammensetzung zur Wirkstoffabgabe nach Anspruch 1, die ferner ein zusätzliches Lösungsmittel umfasst.

5. Zusammensetzung zur Wirkstoffabgabe nach Anspruch 4, wobei das Lösungsmittel ein wässriges Lösungsmittel, ein mit Wasser mischbares Lösungsmittel oder eine Mischung davon ist.

6. Zusammensetzung zur Wirkstoffabgabe nach Anspruch 1, die ferner ein oder mehrere Additive umfasst, ausgewählt aus einem pH-Regulator, einem anorganischen Salz, einem Saccharid, einem Tensid, einem Chelatbildner oder einer Kombination davon.

7. Zusammensetzung zur Wirkstoffabgabe nach Anspruch 1, bestehend aus: einer kationischen Verbindung; einem Salz der Polymilchsäure; einem Lösungsmittel; und einem oder mehreren Additiven, ausgewählt aus einem pH-Regulator, einem anorganischen Salz, einem Saccharid, einem Tensid, einem Chelatbildner oder einer Kombination davon.

8. Zusammensetzung zur Wirkstoffabgabe nach Anspruch 1, wobei die Menge des Salzes der Polymilchsäure 0,1 bis 15 Gewichtsteile, bezogen auf 1 Gewichtsteil der kationischen Verbindung, beträgt.

9. Zusammensetzung zur Wirkstoffabgabe nach Anspruch 1, wobei die Menge des Salzes der Polymilchsäure 0,5 bis 3 Gewichtsteile, bezogen auf 1 Gewichtsteil der kationischen Verbindung, beträgt.

10. Zusammensetzung zur Wirkstoffabgabe nach Anspruch 1, wobei die kationische Verbindung und das Salz der Polymilchsäure zur Herstellung der Nanopartikel in Form einer ein- oder mehrmals gefilterten Lösung verwendet werden.

## Revendications

1. Composition pour l'administration de médicament comprenant des nanoparticules,
dans laquelle les nanoparticules comprennent un composé cationique et un sel d'acide polylactique, et
dans laquelle les nanoparticules ne comprennent pas de polymère amphiphile et de médicament.

2. Composition pour l'administration de médicament selon la revendication 1, dans laquelle la composition est destinée à l'administration intracellulaire de médicament.

3. Composition pour l'administration de médicament selon la revendication 2, dans laquelle le médicament est choisi parmi un acide nucléique, un polypeptide, un virus ou une combinaison de ceux-ci.

4. Composition pour l'administration de médicament selon la revendication 1, comprenant en outre un solvant supplémentaire.

5. Composition pour l'administration de médicament selon la revendication 4, dans laquelle le solvant est un solvant aqueux, un solvant miscible à l'eau ou un mélange de ceux-ci.

6. Composition pour l'administration de médicament selon la revendication 1, comprenant en outre un ou plusieurs additifs choisis parmi un agent d'ajustement du pH, un sel inorganique, un saccharide, un tensioactif, un agent chélatant ou une combinaison de ceux-ci.

7. Composition pour l'administration de médicament selon la revendication 1, constituée de : un composé cationique ; un sel d'acide polylactique ; un solvant ; et un ou plusieurs additifs choisis parmi un agent d'ajustement du pH, un sel inorganique, un saccharide, un tensioactif, un agent chélatant ou une combinaison de ceux-ci.

8. Composition pour l'administration de médicament selon la revendication 1, dans laquelle la quantité de sel d'acide polylactique est de 0,1 à 15 parties en poids, pour 1 partie en poids du composé cationique.

9. Composition pour l'administration de médicament selon la revendication 1, dans laquelle la quantité de sel d'acide polylactique est de 0,5 à 3 parties en poids, pour 1 partie en poids du composé cationique.

10. Composition pour l'administration de médicament selon la revendication 1, dans laquelle le composé cationique et le sel d'acide polylactique sont utilisés pour la préparation des nanoparticules sous la forme d'une solution filtrée une ou plusieurs fois.
